# EUROPEAN PATENT SPECIFICATION

(11) **EP 0 763 737 B1**
(45) Date of publication and mention of the grant of the patent: **15.03.2000**
(21) Application number: 96305901.9
(22) Date of filing: 12.08.1996
(51) Int. Cl.: G01N 33/53, G01N 33/571

(54) **Assays for lipopolysaccharide antigens**
Test für lipopolysaccharide Antigene
Essai pour des antigènes lipopolysaccharides

(30) Priority: 14.09.1995 EP 95306506
(43) Date of publication of application: 19.03.1997
(73) Proprietor: UNIPATH LIMITED, Basingstoke, Hampshire RG24 OPW (GB)
(72) Inventor: Menton, Jeanette Elizabeth Bridget, Bedford, MK40 2JE (GB); Sheard, Paul Raymond, Wilby, Northampton, NN8 2UB (GB)
(74) Representative: Butler, David John

(56) References cited:
- WO-A-89/07765
- WO-A-89/08262
- WO-A-90/02336
- WO-A-94/05166
- US-A- 5 389 547

## Description

### FIELD OF THE INVENTION

This invention relates to assays, and in particular to methods for preparing aqueous samples for use in assays for lipopolysaccharide antigens.

### BACKGROUND TO THE INVENTION

Lipopolysaccharide antigens are important indicators of the presence of certain pathogenic bacteria, notably Chlamydia. Such assays require the preparation of an aqueous extract from a clinical sample suspected of containing the organisms. The sample may, for example, be in the form of a clinical swab taken from a patient. An extraction buffer is used to free lipopolysaccharide antigen from organisms that may be present in the sample. A typical extraction buffer will contain lysing agents such as detergents. The extraction procedure may involve heating of the sample in the extraction buffer, for example to a temperature in the range of 80 to 100°C. After several minutes a sufficient quantity of lipopolysaccharide antigen will have been released from any organisms present to provide basis for an accurate assay.

It is already well known that clinical samples often contain irrelevant components which may interfere with the sensitivity of a lipopolysaccharide antigen assay. Cations are a particular nuisance. EP-A-392865 discusses this problem and recommends that divalent cations should be removed from the system, for example, by chelation with agents such as EDTA. EP-A-392865 is particularly concerned with the unwanted presence of divalent cations such as zinc and magnesium. Other cationically-charged materials may also be present in the clinical sample. Any of these cationic materials may interfere with the sensitivity of a lipopolysaccharide antigen assay. We believe that this interference arises because the cations bind to a negatively charged region of the lipopolysaccharide molecule and block an important binding site against which antibodies useful in such an assay are raised.

### GENERAL DESCRIPTION OF THE INVENTION

By the invention we have found that a substantial reduction in interference caused by cations during a lipopolysaccharide antigen assay can be brought about by using an anionic polysaccharide.

The invention provides an assay for lipopolysaccharide antigen, conducted in the presence of an anionic polysaccharide in an amount sufficient to reduce interference caused by cations.

Preferably the anionic polysaccharide is a glycosaminoglycan, such as heparin. Alternatives include heparin sulphate and dermatan sulphate.

The invention is particularly applicable when the lipopolysaccharide antigen is bacterial, e.g. when the bacteria are Chlamydia.

The invention also provides an aqueous extraction buffer for solubilising antigenic material in bacteria and the like, containing an anionic polysaccharide in an amount of 0,1 mg/ml to 10 mg/ml to enhance assay sensitivity and a surface active agent selected from 3-(3 chlolamidopropyl)di-methylammonio-1-propane sulfonate (CHAPS) or 3(-3 chlolamidopropyl) dimethylammonio-2-hydroxyl-1-propane sulfonate (CHAPSO) or mixtures thereof.

An important embodiment of the invention is this aqueous extraction buffer for use in solubilising lipopolysaccharide antigen in Chlamydia, containing a glycosoaminoglycan, especially heparin, in an amount sufficient to enhance assay sensitivity. Preferably the buffer contains at least about 0.1 mg/ml heparin. Normally it does not need to contain more than about 10 mg/ml heparin.

Another embodiment of the invention is a method of preparing an aqueous assay sample from clinical material suspected of containing micro-organisms, such as Chlamydia, which express lipopolysaccharide antigen, wherein the clinical material or an aqueous extract thereof is contacted with an anionic polysaccharide, especially a glycosoaminoglycan such as heparin, in insolubilised form.

In one embodiment, the invention provides a procedure for extracting solubilised antigenic material from cellular biological material, such as bacteria, wherein the cellular material is treated with an aqueous solution of a surface active agent, in the presence of an anionic polysaccharide. Preferably the surface active agent is zwitterionic. More preferably, the surface active agent is 3-(3-chlolamidopropyl)dimethylammonio-1-propanesulfonate (conveniently known as CHAPS) or 3-(3-chlolamidopropyl)dimethylammonio-2-hydroxyl-1-propanesulfonate(conveniently known as CHAPSO), or mixtures thereof.

An especially effective extraction of lipopolysaccharide antigen from Chlamydia species such as Chlamydia trachomitis, C.psittaci and C.twar, is achieved if the extraction is performed using an aqueous solution of heparin and a zwitterionic surface active agent, especially CHAPS and/or CHAPSO in the presence of an anionic polysaccharide.

Preferably, the extraction is conducted at elevated temperature, for example in excess of about 50°C, for a period of time sufficient to solubilise the antigenic material. More preferably, the extraction temperature is at least about 60°C. In general, the extraction temperature need not be greater than about 100°C, and is preferably not greater than about 90°C. Ideally, the extraction temperature is about 80°C. The stage of the extraction conducted at such elevated temperature should generally last for at least about 5 minutes.

Preferably, the quantity of surface active agent in the aqueous extraction medium is at least about 0.1% by weight. Preferably the quantity of surface active agent is not greater than about 2%, and more preferably not greater than about 1% by weight.

The pH of the extraction medium should generally be in the range of about 7.5 to about 9.

The heparin or other anionic polysaccharies can be used either in soluble or insoluble form as desired. Heparin is available commercially in either form. One option is to have the anionic polysaccharide as a soluble component in the extraction buffer or added as a soluble component at a later stage before commencement of the actual assay procedure. Presence as a component in an extraction buffer is probably the most convenient from the users point of view. Alternatively, an aqueous sample/extract can be contacted with an insolubilised form of the anionic polysaccharide, for example, heparin bound to a solid surface such as resin beads. The insolubilised anionic polysaccharide can be removed from the aqueous composition before the actual assay is conducted. For example beads can be centrifuged or filtered out. A solid phase of greater bulk, for example a high surface area dipstick on which the heparin is bound can be separated from the aqueous composition by manual extraction or decantation of the liquid. A variety of alternative formats will suggest themselves readily to the skilled reader. The essential objective is that the aqueous composition containing the lipopolysaccharide antigens to be assayed is exposed to the anionic polysaccharide under circumstances which permit the anionic polysaccharide to bind with and in effect, remove from the system at least a proportion of any cationic components present which might interfere with the accuracy of the lipopolysaccharide antigen assay. For any given assay procedure and format the optimum amount and presentation of the anionic polysaccharide can be determined by simple experiment.

In a typical extraction procedure according to the invention, a biological sample obtained from a patient suspected of carrying a Chlamydia infection, for example, is contacted with the extraction medium. Appropriate samples can take the form, for example, of genital, rectal or ocular swabs, or centrifugal pellets from liquids such as early morning urine. Extraction, for example at 80°C for 10 minutes, is followed by a brief period, for example 5 minutes, during which the extraction medium is allowed to cool. Thereafter the extraction medium can be separated from solid matter, for example by removal of the swab and filtration of the solution to provide a sample liquid containing any extracted antigen ready for use in any suitable assay procedure. The subsequent assay can involve any conventional assay technique, such as radioimmunoassay or enzyme-linked immunoassay. The extracted sample is ideal for use in an immunochromatographic assay procedure such as described and claimed in EP-A-291194, especially using a nitrocellulose solid phase and an antibody reagent labelled with a direct particulate label such as coloured latex particles. The use of heparin together with CHAPS and/or CHAPSO enhances the sensitivity of a Chlamydia assay which involves anti-lipopolysaccharide antigen antibodies as specific binding reagents.

### EXAMPLES

### Example 1

An extraction procedure for Chlamydia trachomatis can be performed as follows. This provides an extract suitale for use in an immunoassay.

The extraction procedure utilises:
a) Disposable, flexible plastics "test tubes" each capable of holding a volume (eg. 5 ml) of liquid and of accommodating the end of a conventional sampling swab.
b) A heating block in which such tubes can be inserted to permit the contents of the tube to be heated to a temperature in the range 50 - 100°C and held at that temperature for at least a number of minutes.
c) Means for filtering the liquid contents of the tube at the end of the extraction procedure. Conveniently this can take the form of a filter plug incorporated in a perforated stopper with which the tube can be closed and through which the liquid contents can be expelled.
d) An extraction buffer having the following formulation:
   0.25 M TRIS pH 8.5 containing
   0.85% Sodium chloride
   0.25% CHAPSO
   5 mM EDTA
   1% Bovine serum albumin
   1mg/ml Heparin (SIGMA)

To conduct the extraction, a pre-determined quantity (for example 600 µl) of extraction buffer is placed in a tube. A genital swab from a patient suspected of carrying a Chlamydia infection is placed in the tube, and the tube and contents are then incubated in the heating block at a temperature of approximately 80°C for 10 minutes. The tube is removed from the heating block and allowed to cool for 5 minutes. The swab is lifted out of the extraction buffer and, before the swab is removed completely from the tube, the sides of the tube are pressed in gently by hand to squeeze liquid from the swab. The swab can then be removed completely from the tube and discarded. A perforated stopper containing a filter plug is inserted in the top of the tube, and the liquid contents of the tube can be expelled therethrough to provide an essentially clear liquid sample containing any extracted Chlamydia lipopolysaccharide antigen for use in a subsequent assay. If desired, one or more assay reagents, such as labelled antibodies, can be added to the contents of the tube before the filter/stopper is applied to the tube.

The assay can be conducted using any established procedure. Preferably this is accomplished by means of a particle-labelled reagent becoming bound, in a sandwich or competition format assay in the presence of lipopolysaccharide antigen, to reveal the result in a small detection zone, e.g. narrow line, on a porous carrier material such as a nitrocellulose strip, as generally described in EP-A-291194.

### Example 2

The following experiments demonstrate the ability of heparin, either in soluble or insolubilised form, to enhance the sensitivity of a Chlamydia lipopolysaccharide antigen assay.

### Materials and Conditions

### Materials used:

Chlamydia negative female endocervical swabs were washed in saline, washes from five swabs were then pooled. New swab pools were made for each experiment.

Protamine (a small protein molecule) was used to simulate clinical sample interference, at 0.001mg/ml.

Heparin resin (SIGMA H-5380, Lot No. 61 H 9570) was used as a method for removing clinical interferences. Slurries were made using 50% resin to 50% water. 250µl of slurry was centrifuged at 13500rpm for 5 minutes, the supernatant removed and 300µl sample added. Slurry was then mixed end-over-end for 5 minutes. The mixture was centrifuged at 13500 rpm for 5 minutes. The supernatant was then assayed as detailed below.

Extraction buffer (as in Example 1) with and without 1mg/ml soluble heparin (SIGMA).

Procedure: Heat sample in a plastics extraction tube, with filter/dropper cap, at 80°C ± 2°C for 10 minutes. Allow to cool to room temperature for 5 minutes. Place filter cap on extraction tube, and squeeze 5 drops from tube onto a commercially-available "CLEARVIEW Chlamydia" (Unipath Ltd, UK) assay device, as broadly described in EP-A-291194. This uses blue-coloured latex (polystyrene) particles carrying anti-lipopolysaccharide antibodies as a mobile reagent, and anti-LPS antibodies immobilised in a test line as a capture reagent, on a nitrocellulose strip. The assay result is revealed as visible line in test window. Line intensity was assessed optically in arbitrary units, as a measure of antigen detection relative to a standardised control sample.

### Experiment 1

Demonstrating the removal by heparin resin of cationic interferences from clinical samples.

Resin-treated swab pool and untreated swab pool were added to extraction buffer in the presence of a known amount of Chlamydia antigen.
a) 50µl swab pool + 15µl antigen + 535µl extraction buffer
   = 3.5 units (62%)
b) 50µl resin-treated swab pool + 15µl antigen + 535µl extraction buffer
   = 6.0 units (105%)
c) 15µl antigen + 585µl extraction buffer (control)
   = 5.7 units (100%)

### Experiment 2

Demonstrating the ability of soluble heparin to inhibit interferences present in clinical samples.

Swab pool was then added to extraction buffer with and without dissolved heparin, in the presence of a known amount of Chlamydia antigen.
a) 50µl swab pool + 15µl antigen + 535µl extraction buffer (no heparin)
   = 9.9 units (65%)
b) 50µl swab pool + 15µl antigen + 535µl extraction buffer with heparin
   = 13.3 units (88%)
c) 15µl antigen + 585µl extraction buffer (control)
   = 15.1 units (100%)

### Experiment 3

Demonstrating the ability of dissolved heparin or heparin resin to inhibit interferences present in clinical samples.

Resin-treated swab pool and untreated swab pool were then added to extraction buffer (no heparin) with a known amount of Chlamydia antigen.

The untreated swab pool was also tested using extraction buffer with dissolved heparin.
a) 25µl swab pool + 15µl antigen + 560µl extraction buffer = 3.54 units (67%)
b) 25µl swab pool + 15µl antigen + 560µl extraction buffer with heparin = 6.14 units (116%)
c) 25µl of resin-treated swab pool + 15µl antigen + 560µl extraction buffer = 6.49 units (122%)
d) 15µl antigen + 585µl extraction buffer (control) = 5.31 units (100%)

### Experiment 4

Demonstrating the ability of heparin (irrespective of its form) to inhibit interferences present in clinical samples.

0.001mg/ml protamine was used to simulate clinical sample interferences. Soluble heparin and insoluble heparin beads were used.
a) 25µl protamine + 25µl antigen + 950µl extraction buffer (no heparin) = 3.23 units (24.6%)
b) 25µl protamine + 25µl antigen + 100µl heparin beads + 835µl extraction buffer (no heparin) = 11.12 units (84.7%)
c) 25µl protamine + 25µl antigen + 950µl extraction buffer with heparin = 12.63 units (96.2%)
d) 25µl antigen + 950µl extraction buffer (no heparin) = 13.13 units (100%)

## Claims

1. A method of preparing an aqueous assay sample from clinical material suspected of containing a micro-organism that expresses lipopolysaccharide antigen, wherein said clinical material is treated with an aqueous extraction medium effective to release lipopolysaccharide antigen from said micro-organism if present and provide an aqueous extract characterised in that said clinical sample or said aqueous extract is contacted with an anionic polysaccharide in an amount sufficient to reduce assay interference caused by cations.

2. A method according to claim 1, characterised in that said clinical sample is a genital swab, a rectal swab, an ocular swab or a centrifugal pellet from urine.

3. A method according to claim 1 or claim 2, characterised in that said clinical sample or said aqueous extract is contacted with said anionic polysaccharide in insolubilized form.

4. A method according to claim 1 or claim 2, characterised in that said anionic polysaccharide is a constituent of said aqueous extraction medium.

5. A method according to any one of the preceding claims, characterised in that said anionic polysaccharide is a glycosaminoglycan.

6. A method according to claim 5, characterised in that said glycosaminoglycan is heparin.

7. A method according to claim 6, characterised in that said aqueous extraction medium contains at least about 0.1 mg/ml heparin.

8. A method according to claim 5, characterised in that said glycosaminoglycan is heparin sulphate or dermatan sulphate.

9. A method according to any one of the preceding claims, characterised in that said micro-organism is a species of Chlamydia.

10. A method according to claim 9, characterised in that said aqueous extraction medium contains a surface active agent selected from the group consisting of 3-(3-chlolamidopropyl) dimethylammonio-1-propanesulfonate and 3-(3-chlolamidopropyl) dimethylammonio-2-hdroxyl-1-propanesulfonate and mixtures thereof, in an amount sufficient to release lipopolysaccharide antigen from cells of Chlamydia trachomatis.

11. A method according to claim 10, characterised in that said aqueous extraction medium contains said anionic polysaccharide.

12. A method according to claim 11, characterised in that said aqueous extraction medium contains from about 0.1 to about 10 mg/ml heparin.

13. An aqueous solution containing a surface active agent selected from the group consisting of 3-(3-chlolamidopropyl) dimethylammonio-1-propanesulfonate and 3-(3-chlolamidopropyl) dimethylammonio-2-hydroxyl-1-propanesulfonate and mixtures thereof, in an amount sufficient to release lipopolysaccharide antigen from cells of Chlamydia trachomatis, characterised in that said aqueous solution contains from 0.1 to 10 mg/ml of an anionic polysaccharide.

14. An aqueous solution according to claim 13, characterised in that said anionic polysaccharide is a glycosaminoglycan.

15. An aqueous solution according to claim 14, characterised in that said glycosaminoglycan is heparin.

## Revendications

1. Procédé de préparation d'un échantillon aqueux de dosage à partir d'une matière clinique susceptible de contenir un micro-organisme qui exprime un antigène lipopolysaccharide, dans lequel ladite matière clinique est traitée avec un milieu aqueux d'extraction efficace pour libérer l'antigène lipopolysaccharide éventuel dudit micro-organisme et pour fournir un extrait aqueux, caractérisé en ce que ledit échantillon clinique ou ledit extrait aqueux est mis en contact avec un polysaccharide anionique en une quantité suffisante pour réduire l'interférence de dosage provoquée par les cations.

2. Procédé selon la revendication 1, caractérisé en ce que ledit échantillon clinique est un prélèvement génital, un prélèvement rectal, un prélèvement oculaire ou une pastille centrifugée d'urine.

3. Procédé selon la revendication 1 ou la revendication 2, caractérisé en ce que ledit échantillon clinique ou ledit extrait aqueux est mis en contact avec ledit polysaccharide anionique sous forme insolubilisée.

4. Procédé selon la revendication 1 ou la revendication 2, caractérisé en ce que ledit polysaccharide anionique est un constituant dudit milieu aqueux d'extraction.

5. Procédé selon l'une quelconque des revendications précédentes, caractérisé en ce que ledit polysaccharide anionique est un glycosaminoglycane.

6. Procédé selon la revendication 5, caractérisé en ce que ledit glycosaminoglycane est l'héparine.

7. Procédé selon la revendication 6, caractérisé en ce que ledit milieu aqueux d'extraction contient au moins environ 0,1 mg/ml d'héparine.

8. Procédé selon la revendication 5, caractérisé en ce que ledit glycosaminoglycane est le sulfate d'héparine ou le sulfate de dermatane.

9. Procédé selon l'une quelconque des revendications précédentes, caractérisé en ce que ledit micro-organisme est une espèce de type Chlamydia.

10. Procédé selon la revendication 9, caractérisé en ce que ledit milieu aqueux d'extraction contient un tensioactif choisi dans le groupe constitué par le diméthylammonio-1-propanesulfonate de 3-(3-chlolamidopropyle) ou le diméthylammonio-2-hydroxyl-1-propanesulfonate de 3-(3-chlolamidopropyle) ou des mélanges de ceux-ci, en une quantité suffisante pour libérer un antigène lipopolysaccharide des cellules de Chlamydia trachomatis.

11. Procédé selon la revendication 10, caractérisé en ce que ledit milieu aqueux d'extraction contient ledit polysaccharide anionique.

12. Procédé selon la revendication 11, caractérisée en ce que ledit milieu aqueux d'extraction contient d'environ 0,1 à environ 10 mg/ml d'héparine.

13. Solution aqueuse contenant un tensioactif choisi dans le groupe constitué par le diméthylammonio-1-propanesulfonate de 3-(3-chlolamidopropyle) ou le diméthylammonio-2-hydroxyl-1-propanesulfonate de 3-(3-chlolamidopropyle) et des mélanges de ceux-ci, en une quantité suffisante pour libérer un antigène lipopolysaccharide des cellules de Chlamydia trachomatis, caractérisée en ce que ladite solution aqueuse contient de 0,1 à 10 mg/ml d'un polysaccharide anionique.

14. Solution aqueuse selon la revendication 13, caractérisée en ce que ledit polysaccharide anionique est un glycosaminoglycane.

15. Solution aqueuse selon la revendication 14, caractérisée en ce que ledit glycosaminoglycane est l'héparine.

## Patentansprüche

1. Verfahren zur Herstellung einer flüssigen Assayprobe aus klinischem Material, das mutmaßlich einen Lipopolysaccharidantigen expremierenden Mikroorganismus enthält, wobei das klinische Material mit einem wäßrigen Extraktionsmedium behandelt wird, das zum Herauslösen des Lipopolysaccharidantigens aus dem Mikroorganismus, falls vorhanden, und zum Bereitstellen eines wäßrigen Extraktes geeignet ist, dadurch gekennzeichnet, daß die klinische Probe oder der wäßrige Extrakt mit dem anionischen Polysaccharid in einer Menge in Kontakt gebracht wird, die zur Reduktion von durch Kationen verursachten Assaystörungen ausreicht.

2. Verfahren gemäß Anspruch 1, dadurch gekennzeichnet, daß die klinische Probe ein genitaler Abstrich, ein rektaler Abstrich, ein okularer Abstrich oder ein zentrifugiertes Pellet aus Urin ist.

3. Verfahren gemäß Anspruch 1 oder Anspruch 2, dadurch gekennzeichnet, daß die klinische Probe oder der wäßrige Extrakt mit dem anionischen Polysaccharid in unlöslich gemachter Form in Kontakt gebracht wird.

4. Verfahren gemäß Anspruch 1 oder Anspruch 2, dadurch gekennzeichnet, daß das anionische Polysaccharid ein Bestandteil des wäßrigen Extraktionsmediums ist.

5. Verfahren gemäß mindestens einem der vorhergehenden Ansprüche, dadurch gekennzeichnet, daß das anionische Polysaccharid ein Glucosaminoglycan ist.

6. Verfahren gemäß Anspruch 5, dadurch gekennzeichnet, daß das Glucosaminoglycan Heparin ist.

7. Verfahren gemäß Anspruch 6, dadurch gekennzeichnet, daß das wäßrige Extraktionsmedium mindestens etwa 0,1 mg/ml Heparin enthält.

8. Verfahren gemäß Anspruch 5, dadurch gekennzeichnet, daß das Glucosaminoglycan Heparinsulfat oder Dermatansulfat ist.

9. Verfahren gemäß mindestens einem der vorhergehenden Ansprüche, dadurch gekennzeichnet, daß der Mikroorganismus eine Chlamydia Spezies ist.

10. Verfahren gemäß Anspruch 9, dadurch gekennzeichnet, daß das wäßrige Extraktionsmedium ein oberflächenaktives Mittel enthält, das ausgewählt ist aus der Gruppe, die aus 3-(3-Cholanamidopropyl)-dimethylammonio-1-propansulfonat und 3-(3-Cholanamidopropyl)-dimethylammonio-2-hydroxyl-1-propansulfonat und Mischungen aus diesen besteht, in einer zum Herauslösen des Lipopolysaccharidantigens aus den Zellen von Chlamydia trachomatis ausreichenden Menge.

11. Verfahren gemäß Anspruch 10, dadurch gekennzeichnet, daß das wäßrige Extraktionsmedium das anionische Polysaccharid enthält.

12. Verfahren gemäß Anspruch 11, dadurch gekennzeichnet, daß das wäßrige Extraktionsmedium etwa 0,1 bis etwa 10 mg/ml Heparin enthält.

13. Wäßrige Lösung, enthaltend ein aus der aus 3-(3-Cholanamidopropyl)-dimethylammonio-1-propansulfonat und 3-(3-Cholanamidopropyl)-dimethylammonio-2-hydroxyl-1-propansulfonat und Mischungen aus diesen bestehenden Gruppe ausgewähltes oberflächenaktives Mittel, in einer zum Herauslösen von Polysaccharidantigen aus Zellen von Chlamydia trachomatis ausreichenden Menge, dadurch gekennzeichnet, daß die wäßrige Lösung 0,1 bis 10 mg/ml eines anionischen Polysaccharids enthält.

14. Wäßrige Lösung gemäß Anspruch 13, dadurch gekennzeichnet, daß das anionische Polysaccharid ein Glucosaminoglycan ist.

15. Wäßrige Lösung gemäß Anspruch 14, dadurch gekennzeichnet, daß das Glucosaminoglycan Heparin ist
